# EUROPEAN PATENT SPECIFICATION

(11) **EP 4 441 492 B1**
(45) Date of publication and mention of the grant of the patent: **25.11.2026**
(21) Application number: 22834720.9
(22) Date of filing: 01.12.2022
(51) Int. Cl.: G01N 22/00, G01N 33/483, G01N 33/487

(54) **METHOD AND RELATIVE SYSTEM FOR THE DETECTION OF A VIRAL AGENT BY MICROWAVE DIELECTRIC SPECTROSCOPY**
VERFAHREN UND SYSTEM ZUM NACHWEIS EINES VIRUSMITTELS MITTELS DIELEKTRISCHER MIKROWELLENSPEKTROSKOPIE
PROCÉDÉ ET SYSTÈME ASSOCIÉ POUR LA DÉTECTION D'UN AGENT VIRAL PAR SPECTROSCOPIE DIÉLECTRIQUE À MICRO-ONDES

(30) Priority: 02.12.2021 IT 202100030557
(43) Date of publication of application: 09.10.2024
(73) Proprietor: Istituto Nazionale di Astrofisica, 00136 Roma (RM) (IT); Università degli Studi di Roma "La Sapienza", 00185 Roma (IT); Istituto Superiore di Sanità, 00161 Roma (IT); Consiglio Nazionale Delle Ricerche, 00185 Roma (IT)
(72) Inventor: BOLLI, Pietro, 00136 ROMA (IT); BRUCALASSI, Anna, 00136 ROMA (IT); CHIARUCCI, Simone, 00136 ROMA (IT); CRESCI, Luca, 00136 ROMA (IT); DEL VECCHIO, Ciro, 00136 ROMA (IT); DI NINNI, Paola, 00136 ROMA (IT); NESTI, Renzo, 00136 ROMA (IT); PANELLA, Dario, 00136 ROMA (IT); TOZZI, Andrea, 00136 ROMA (IT); APOLLONIO, Francesca, 00185 ROMA (IT); LIBERTI, Micaela, 00185 ROMA (IT); ARGENTINI, Claudio, 00161 ROMA (IT); AMENDOLA, Antonello, 00161 ROMA (IT); FIORENTINI, Cristiano, 00161 ROMA (IT); FORTUNA, Claudia, 00161 ROMA (IT); MARSILI, Giulia, 00161 ROMA (IT); VENTURI, Giulietta, 00161 ROMA (IT); BARUCCI, Marco, 00185 ROMA (IT)
(74) Representative: Studio Torta S.p.A.
(86) International application number: PCT/IB2022/061649
(87) International publication number: WO 2023/100132

(56) References cited:
- WO-A1-02/46357
- US-A1- 2009 237 067
- US-A1- 2015 355 110
- ANIL LONAPPAN: "Novel method of detecting H1N1 using microwaves", JOURNAL OF BIOMEDICAL SCIENCE AND ENGINEERING, vol. 05, no. 08, 1 January 2012 (2012-01-01), pages 476 - 479, XP055336771, ISSN: 1937-6871, DOI: 10.4236/jbise.2012.58060
- CHIA-FENG LIU ET AL: "Single cell impedance analysis and electrical characterization in micro-fluidic device", NANO/MICRO ENGINEERED AND MOLECULAR SYSTEMS (NEMS), 2011 IEEE INTERNATIONAL CONFERENCE ON, IEEE, 20 February 2011 (2011-02-20), pages 121 - 126, XP031965865, ISBN: 978-1-61284-775-7, DOI: 10.1109/NEMS.2011.6017310

## Description

### Cross-Reference to Related Applications

This Patent Application claims priority from Italian Patent Application No.102021000030557filed on December 2, 2021.

### Technical Field

The present invention concerns a method and the relative system for the detection of a viral agent by applying the dielectric spectroscopy technique in the microwave frequency band. The invention has applications in the detection of viral agents, including the SARS-CoV-2 virus, dispersed in an isotonic solution.

### Background

The current health emergency caused by the global and uncontrolled spread of the SARS-CoV-2 virus, has highlighted the fundamental role played by diagnostic techniques, as tools that effectively contribute to countering the spread of the pandemic. In fact, thanks to the timely and accurate detection of the presence of a specific viral agent in a subject, it is possible to implement targeted and timely actions starting from isolating the individual who tested positive for viral infection and, if necessary, from administering an appropriate drug therapy.

Laboratory tests of biological samples (e.g. nasopharyngeal swab) are those that are most likely to detect the presence of viral material promptly, effectively and to a large extent and thus provide a practically certain diagnosis of viral infection.

To date, the most commonly used laboratory diagnostic tests are those analysing biological samples by polymerase chain reaction (PCR) tests and, more precisely, by "real time" PCR. "Real time" PCR is a molecular biology technique that simultaneously amplifies fragments of viral nucleic acids (directly DNA or DNA starting from an RNA template) and quantifies DNA. The amplified DNA sample is analysed and quantified via emission or absorption spectroscopic techniques, thanks to the introduction of fluorescent markers during the reaction step that bind to the amplified DNA. However, these detection techniques have important limitations such as the minimum concentration of biological material to be analysed for the faster techniques or the analysis time for the most significant quantitative ones. In addition, these techniques based on the detection of viral nucleic acid must be performed in specialised laboratories and by highly qualified operators and require long time for the preparation of the biological sample to be subjected to analysis. It is therefore evident that under conditions of high diagnostic demand, such as during a pandemic emergency, molecular techniques alone are not sufficient.

Given the limitations associated with the PCR-based techniques, alternative methodologies for the detection of viral agents are being developed and some of them are already commercially available. Among those already in clinical use there are antigenic tests, which detect the presence of viral proteins capable of binding to antibodies, and serological tests, which detect the presence of antibodies corresponding to a certain antigen in the patient's blood plasma to ascertain whether exposure to a certain pathogen occurred.

Among the techniques under development there are those employing:
- mass spectrometry, on the basis of which the presence of viral proteins/peptides is detected,
- biosensors capable of detecting proteins and viral RNA,
- microscopy that adopts fluorescent markers and machine learning to identify viral particles,
- the tests that are carried out on the breath given by a subject, analysing biomarkers such as volatile organic compounds,
- skin tests, by means of which it is possible to detect changes in the amount of lipids circulating in the blood (dyslipidemia), resulting from viral infections, which occur at the level of the skin.

However, the performance of the diagnostic techniques under development mentioned above are still uncertain both in terms of sensitivity and specificity and not yet validated by the regulatory authorities.

As an alternative to recent diagnostic solutions for the detection of viral agents, one can also include those techniques exploiting the potential of microwave dielectric spectroscopy.

To date, microwave dielectric spectroscopy is a well-established discipline that uses electromagnetic radiations in the microwave band to derive the dielectric parameters of a sample under test. For instance, studies are known to exploit microwaves to detect structural information of cells in biological solutions, as well as applications of dielectric spectroscopy for the detection of molecular components such as glucose and nanometric particles such as liposomes.

A microwave-based method for detecting the H1N1 viral agent is disclosed in A. Lonappan, "Novel method of detecting H1N1 using microwaves", JOURNAL OF BIOMEDICAL SCIENCE AND ENGINEERING, vol. 05, no. 08, 1 January 2012.

### Object of the Invention

Aim of the present invention is therefore to provide an alternative method for detecting a viral agent that allows to overcome the limits previously exposed and to guarantee performance, in terms of sensitivity and reproducibility of the results, comparable or even superior to the known techniques already in use or being developed.

In accordance with this aim, the present invention relates to a method according to claim 1.

A further aim of the invention is to provide a detection system usable for such a method.

In accordance with this further object, the present invention is further related to a detection system according to claim 10.

This method therefore makes it possible to exploit the advantages of microwave dielectric spectroscopy which have already been found in a wide range of applications, including in the biological field. In fact, microwave dielectric spectroscopy is a technique that does not require the use of markers and allows samples of interest to be analysed in a non-destructive manner and quickly. Thus, the present method, contrary to the PCR-based diagnostic systems, makes it possible to detect the presence of viral agents in the solution of interest rapidly, without introducing fluorescent markers, without altering the starting solution and also in cases where viral agents are present in low concentrations.

In more detail, this method is based on a differential approach in which on a reference solution firstly a measurement is taken and then a second measurement is taken in which a viral agent is introduced into the reference solution. This method therefore allows the detection of a viral agent by characterising its dielectric properties and, mainly, its relative dielectric permittivity as a function of the frequencies of the electromagnetic field applied to the solution in which the viral agent is dispersed. Since the dielectric properties of a viral agent are strongly linked to its molecular structure, when the electromagnetic radiation passes through the solution of interest, the viral agents dispersed therein interact with the radiation, generating a characteristic signal associated with its intrinsic dielectric properties that differs from the signals associated with the dielectric properties of the solution.

In microwave dielectric spectroscopy, there are several measurement techniques through which it is possible to derive the dielectric properties of the sample of interest: among them, resonant or non-resonant measurement techniques can be distinguished. Resonant measurement techniques allow the dielectric properties of the sample to be characterised in a single frequency with high precision. Whereas the non-resonant techniques allow to derive the dielectric properties of the sample in a relatively wide range of frequencies. The present method adopts a non-resonant measurement technique using the transmission lines to guide the radiation in the microwaves towards the sample, and then to conduct the response signal, reflected and transmitted by the sample itself, towards an analyser. In fact, the measurement technique adopted here is called transmission/reflection technique and allows to measure both the transmission and reflection parameters (in both cases amplitude and phase) on a rather wide frequency band in the microwaves (frequencyₘₐₓ/frequencyₘᵢₙ=1.5); this allows obtaining a very complete information on the dielectric material under test.

In addition, this method advantageously exploits a differential technique between two sequential measures; systematic errors due to the measurement structure (imperfections of the sample or of the sample holder or of the transmission line) and to the measurement instrument (calibration problem) are thus removed, as well as unwanted signals ("noise") present in the measurement circuit.

A further advantage of the present method is attributable to the fact that this method makes it possible to directly derive the intrinsic dielectric properties of the reference solution and, subsequently by comparison, of the viral agent.

### Brief Description of the Drawings

The present invention will now be described with reference to the accompanying drawings, wherein:
Figure 1 is a schematic of a detection system for implementing the experimental steps of the method according to the invention;
Figure 2 is a front view of a sample holder according to the invention;
Figures 3 and 4 are sections along the lines III-III and IV-IV in Figure 2;
Figure 5 is a perspective view of the sample holder of Figure 2;
Figure 6 shows in detail some elements of the detection system of Figure 1;
Figure 7 is a flowchart showing the steps of the method of the present invention;
Figures 8 and 9 are two flowcharts showing the data processing steps of the method of Figure 7 with mathematical models;
Figure 10 qualitatively shows the amplitude of the reflection and transmission scattering parameters in the time domain acquired experimentally in a step of the method according to the invention;
Figures 11 and 12 show the trend of the reflection and transmission scattering parameters, respectively, as the frequency obtained from experimental measures taken using the detection system of Figure 1 varies;
Figure 13 shows the trend of the attenuation constant and of the propagation constants as a function of the frequencies calculated in accordance with the data processing step of Figure 9;
Figures 14 and 15 show the trend of the relative dielectric permittivity εᵣ and of the loss tangent tanδ, respectively, as a function of the frequencies calculated in accordance with the data processing step of Figure 8;
Figures 16 and 17 show the trend of the relative dielectric constant εᵣ and of the loss tangent tanδ, respectively, as a function of the frequencies calculated in accordance with the data processing step of Figure 9;
Figure 18 shows the trend of the real part of the dielectric permittivity as the frequency varies for a reference solution and for a solution containing a viral agent;
Figure 19 shows the difference of the permittivity values, indicated in Figure 18, between the solution containing a viral agent and the reference solution; and
Figures 20 and 21 show two contrasts obtained starting from experimental data acquired during two separate measurement sessions.

### Description of Embodiments

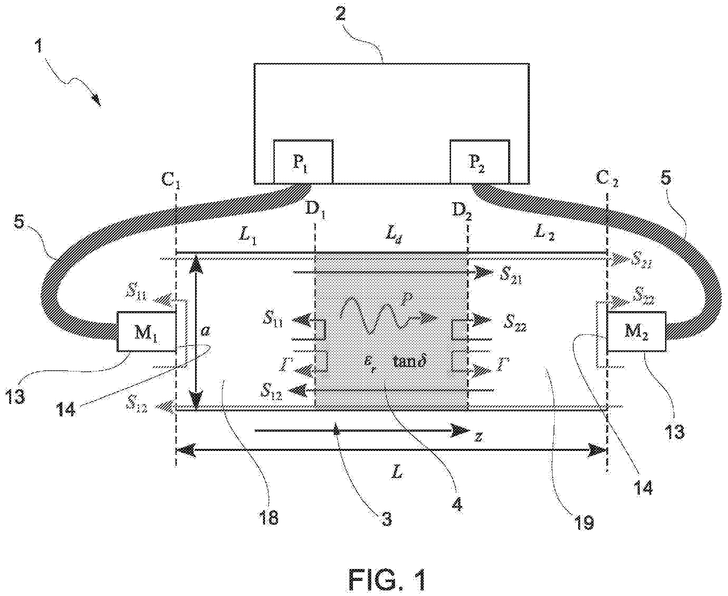

With reference to Figure 1, a detection system 1 is shown, by means of which part of the method according to the invention is implemented.

The detection system 1 comprises a vector network analyser (VNA) 2 equipped with at least two ports (port 1, P1, and port 2, P2), a sample holder 3 adapted to contain a sample 4 to be tested, and two transmission lines 5 connecting the ports P1 and P2 of the VNA 2 to the sample holder 3.

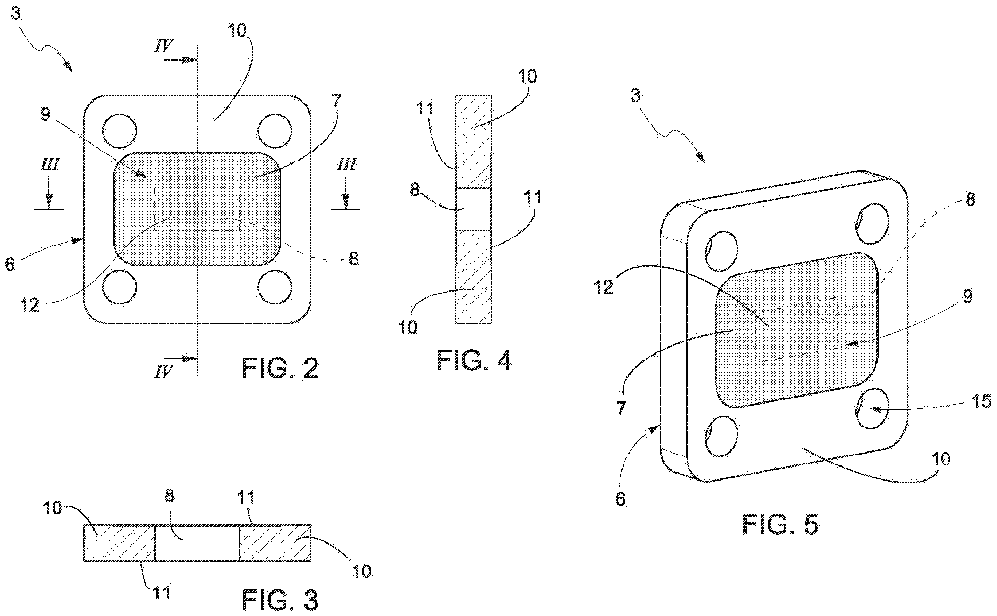

The sample holder 3 is shown in Figures 2 to 5 and comprises a plate 6 having a hollow central portion 7 provided with a rectangular through cavity 8 and defining a waveguide 9 and a fixing perimeter flange 10.
In a preferred embodiment of the sample holder 3, the waveguide 9 has a rectangular cross section, where a is the major side.

Preferably, the waveguide 9 is of the WR28 type (transverse dimensions: 7.1 mm x 3.6 mm) operating in the 26 GHz to 40 GHz frequency band.

The plate 6 has, on opposite faces of the central portion 7, two rectangular seats 11 slightly recessed with respect to the thickness of the flange 10, in which the respective containment elements 12 are applied, for example transparent adhesive hermetic films to the microwaves to axially close the cavity 8 adapted to house the sample, which is therefore confined laterally by the internal surfaces of the cavity 8 and axially by the pair of containment elements 12. The distance between the containment elements 12, i.e. the thickness of the central portion 7 of the plate 6, defines a length Ld of the waveguide 9. The length Ld is chosen so as to house a volume of sample 4 containing a sufficient amount of biological material and still ensure an adequate level of the acquired signal since the attenuation of the signal along the waveguide 9 is of the order of about 20 dB/mm.

In a preferred embodiment of the invention, the sample holder 3 has a length Ld equal to 3 mm, corresponding to a volume of liquid equal to 75 mm³. These dimensions allow a good compromise to be reached between the attenuation level of the detected signal and the amount of solution to be tested.

The ports P1, P2 of the VNA 2 (Figure 1) are connected to the sample holder 3 via the respective transmission lines 5 which are adapted to transmit both the input signals from the VNA 2 to the sample 4 and the output signals from the sample 4 to the VNA 2. The transmission lines 5 are preferably of the coaxial type and are connected to the sample holder 3 by means of respective coaxial cable/waveguide adapters 13 to ensure continuity of propagation of the radiations in the microwaves.

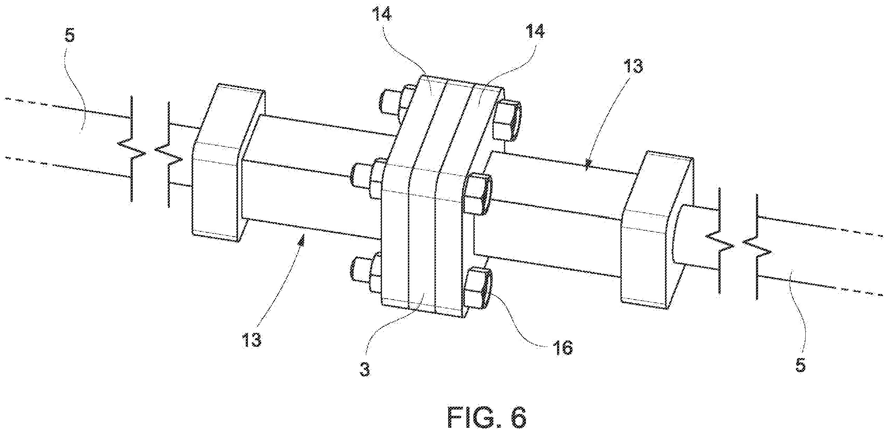

The adapters 13 are equipped with rectangular end flanges 14 fixed to opposite sides of the flange 10 of the sample holder 3. The flanges 10 and 14 are equipped with the aim of holes 15 arranged near their vertices, for mechanical fixing by means of screws 16 (Figure 6).

Thanks to the presence of the seats 11 in the sample holder 3, which house the containment elements 12, a perfect electrical continuity is ensured between the flanges 14 and the flange 10.

Figure 6 shows in detail the portion of the detection system 1 near the sample holder 3. In succession and symmetrically starting from the sample holder 3, the adapters 13 equipped with flanges 14 and the transmission lines 5 are shown.

Returning to Figure 1, although the interface of the VNA is constituted by the ports P1 and P2, the actual interface of the instrument with the sample holder 3 is constituted by the ports indicated with M1 and M2 which are directly connected with the sample holder itself (in practice, defined by the planes C1, C2 of contact between the flanges 14 of the adapters 13 and the sample holder 3). By means of a calibration procedure of known type and not described in detail here, it is possible to filter the effects on the signals produced by the transmission lines 5 and by the adapters 13 so as to "read" only the signals returned by the sample holder 3 as if it were connected directly to the ports P1 and P2 of the VNA 2.

In order to correctly process the acquired experimental signals, it is necessary to consider not only the sample holder 3 of axial length Ld, but also two extra portions 18 and 19 of axial length L1 and L2, respectively, which extend between each of the ports M1 and M2 and the interface sections D1, D2 between the sample 4 and the respective containment elements 12.

The two extra portions 18 and 19 must be treated as two empty waveguide portions that represent the effects on the propagation of the electromagnetic radiation due to electrical and geometric discontinuities of the sample holder 3 such as, for example, the presence of the pair of containment elements 12 and to the relative seats 11. It is therefore more correct to state that the sample holder 3 has an overall axial length L given by the actual length Ld (known) and by the lengths L1 and L2 of the two extra portions 18 and 19 (calculable as described in detail below).

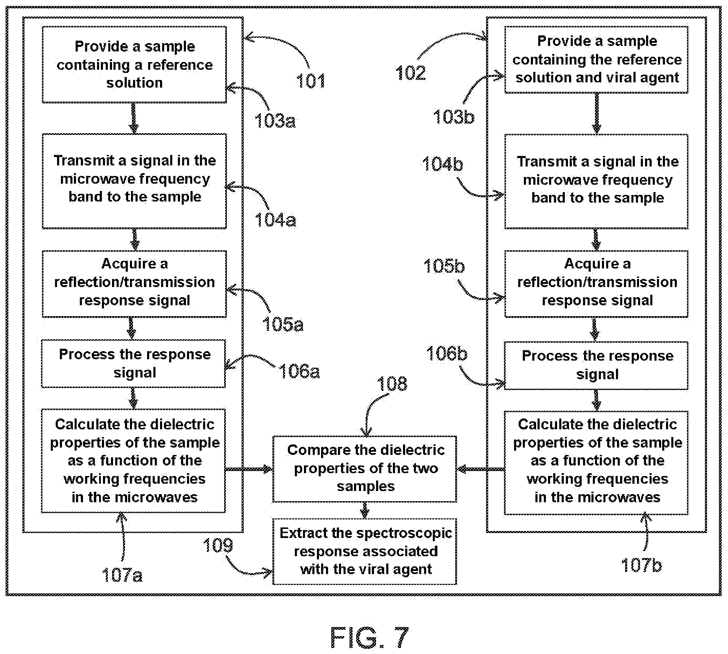

Figure 7 shows a flowchart of a method for detecting a viral agent according to the present invention.

The method comprises, in sequence, a first step of measuring 101 the dielectric properties of a reference sample, a second step of measuring 102 the dielectric properties of a sample under test containing a viral agent, a step 108 of comparing the dielectric properties obtained from the measuring steps 101 and 102 and a step 109 of detecting the viral agent. In turn, the first and second measuring steps comprise respective series of sequential steps (103 to 107) equal to each other and applied respectively to the reference sample and to the sample under test.

In the first measuring step 101 the reference sample is constituted by a reference aqueous solution, preferably an isotonic buffer solution, for example a saline phosphate buffer (PBS) containing disodium hydrogen phosphate (Na₂HPO₄), sodium chloride (NaCl) and, in some formulations, potassium chloride (KCl) and potassium dihydrogen phosphate (KH₂PO₄).

Step 103a of the first measuring step 101 envisages inserting the reference solution into the sample holder 3. The subsequent step 104a comprises the operation of transmitting to the sample 4 signals in the microwave frequency band ranging between 26 and 40 GHz.

In a subsequent step 105a, reflection and transmission response signals are acquired via the VNA 2 from the sample 4.

In the subsequent step 106a, the response signals are processed by post-processing operations described in detail below.

Finally, in step 107a the dielectric properties of the reference sample (mainly, relative dielectric permittivity and loss tangent) are calculated.

Steps 103b-107b of the second measuring step 102 correspond to the described steps 103a-107a, with the only difference that the sample under test is not a reference isotonic solution, but is the same solution comprising a viral agent.

The viral agent used in an experimental step of the present method is a virus-like particle (VLP). In detail, VLPs are engineered virus-like particles that are widely used in many areas of scientific research as they can be manipulated without observing safety protocols as required when treating genuine viruses, i.e. containing viral genetic material. It is generally recognized that VLPs mimic the organization and conformation of genuine viruses. It follows that also the dielectric properties of VLPs are similar to those of the corresponding authentic and infectious viruses. Therefore, the method of the present invention, validated for the detection of VLPs, is also valid for the detection of the corresponding infectious virus.

In a preferred embodiment of the present method, the VLPs used are particles similar to those of the HIV/SIV virus, but absolutely non-infectious as they lack the viral genome. The latter therefore require a level of biosecurity equal to 1. The inner part of the particles is characterised by the nucleus of the SIV virion (SIV-GAG) to which the expressing GFP (Green Fluorescent Protein) gene has been associated. The coating is characterised by the surface protein of HIV modified to form a stable trimer capable of promoting the production of neutralizing antibodies.

The measuring steps 101 and 102 are followed by step 108 of comparing or, more properly, of differential analysis, between the dielectric properties calculated in steps 107a and 107b. The difference between the dielectric properties derived from the first measurement 101 and from the second measurement 102 is indicative of the presence of viral agent. From the differential analysis of step 108 performed in the frequency domain, a specific spectroscopic response associated with the viral agent can be extracted.

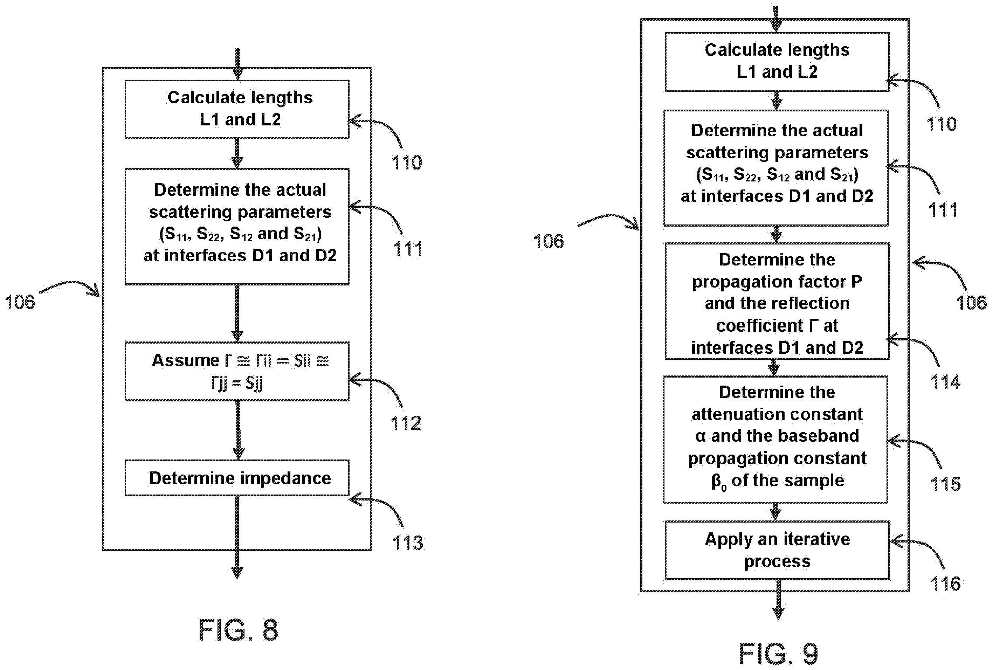

With reference to Figures 8 and 9, the processing steps 106a and 106b are now described, collectively indicated with 106, starting from the reflection (s₁₁ and s₂₂) and transmission (s₂₁ and s₁₂) scattering parameters acquired experimentally in steps 105a and 105b. In short, from the scattering parameters acquired by the VNA, the lengths L1 and L2 of the two extra portions 18 and 19 are derived. The lengths L1 and L2 of the two extra portions 18 and 19 being known, the actual transmission (S₁₁ and S₂₂) and reflection (S₁₂ and S₂₁) scattering parameters associated with the two interfaces D1 and D2 are derived. From them, by applying mathematical models, the propagation factor P in the sample 4, the reflection coefficient Γ at the interfaces D1 and D2, and, finally, the dielectric properties of the sample 4, such as the relative dielectric permittivity εᵣ and the loss tangent tanδ are derived.

The entire mathematical process for calculating the dielectric properties starting from the measured scattering parameters is referred to as "the inverse problem".

Many equations and theoretical aspects that will be presented shortly are part of the theory on the propagation of the electromagnetic waves discussed for example in R. E. Collin, Foundation for Microwave Engineering, Wiley, 2001 and in C. A. Balanis, Advanced Engineering Electromagnetics, Wiley, 1989. The problem of characterization of the dielectric properties of a liquid solution is addressed as a general problem of characterization of a dielectric material in a wave propagation structure, according to the general transmission/reflection method described for example in L. F. Chen et al., Microwave Electronics: Measurements and Materials Characterization, John Wiley & Sons, 2004 and in J. Baker-Jarvis et al. Improved technique for determining complex permittivity with the transmission and reflection method, in IEEE Transactions on Microwave Theory and Techniques, vol. 38, pp. 1096-1103, Aug. 1990.

In Figure 8, step 106 relates to an approximate discussion of the inverse problem, while in Figure 9 step 106 considers the full inverse problem. Steps 110 and 111 are common both to the inverse problem in approximate form and to the full resolution of the inverse problem, and concern the calculation of the lengths L1 and L2 of the two extra portions 18 and 19 and the determination of the actual scattering parameters (S₁₁, S₂₂, S₁₂ and S₂₁) at the interfaces D1 and D2.

In detail, step 110 consists of transforming the scattering parameters (s₁₁, s₂₂, s₁₂ and s₂₁) acquired in the frequency domain into the corresponding scattering parameters in the time domain by applying the inverse discrete Fourier transform.

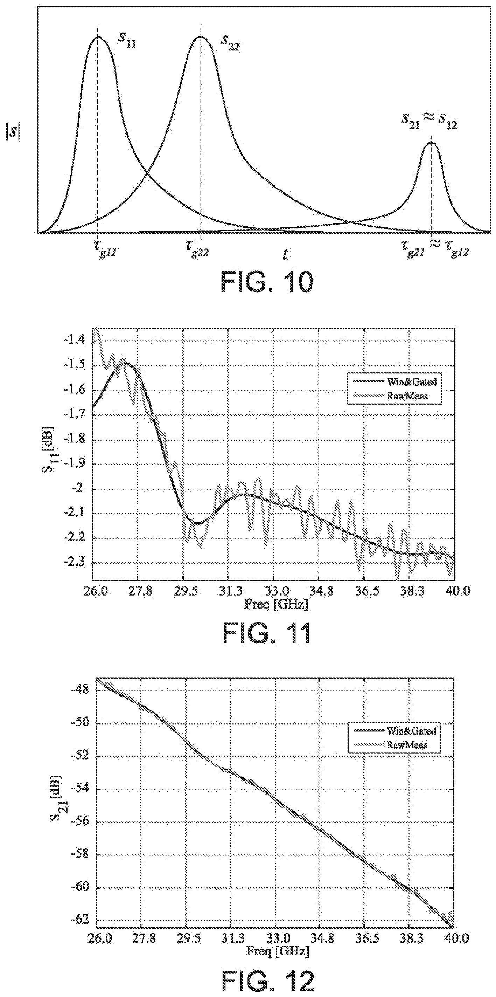

As shown in Figure 10, the curves exhibit peaks associated with the geometric or electric discontinuities along the waveguide, in particular two substantially coincident peaks s₁₁ and s₂₂ corresponding to the position of the interfaces with the liquid, and substantially coincident peaks s₂₁ and s₁₂ corresponding to the total length of the waveguide. The width of the latter is coincident given the reciprocity of the path. Peak delay times are understood as group delays of the waves. As regards s₁₁, the group delay t_{g11} corresponds to the path of the wave back and forth from the port M1 to the interface D1 closest to M1. Thus, t_{g11} corresponds to the time it takes to the wave to propagate back and forth along the length L1 of the extra portion 18. Similarly, the group delay t_{g22} corresponds to the time it takes to the wave to propagate back and forth along the length L2 of the extra portion 19. Taking into account the reflection scattering parameters (s₁₁ and s₂₂) it is therefore possible to calculate the lengths L1 and L2 based on the following relationships: ${L}_{1} = \frac{{τ}_{g 11} {v}_{g}}{2} ; {L}_{2} = \frac{{τ}_{g 22} {v}_{g}}{2}$ (1) wherein *v_{g}* is the group speed in an empty waveguide, having the same section as the waveguide 9.

Given the lengths L1 and L2, it is proceeded with the calculation of the actual scattering parameters with reference to the interfaces D1 and D2 (step 111) based on the relationships: $\left\{\begin{matrix}{S}_{11} = {s}_{11} {e}^{- {jγ}_{a} {L}_{1}} \\ {S}_{21} = {s}_{21} {e}^{- {jγ}_{a} \left({L}_{1}+{L}_{2}\right)} \\ {S}_{12} = {s}_{12} {e}^{- {jγ}_{a} \left({L}_{1}+{L}_{2}\right)} \\ {S}_{22} = {s}_{22} {e}^{- {jγ}_{a} {L}_{2}}\end{matrix}\right.$ (2) wherein *γₐ* is the complex propagation constant in an empty rectangular waveguide of transverse dimensions a and b, wherein a is the largest dimension.

Steps 110 and 111, in Figure 8 are followed by step 112 in which the reflection coefficient Γ is assumed to be equal to the interface D1 and D2 equivalent to the actual reflection scattering parameter (S₁₁ or S₂₂), i.e. $Γ ≅ {Γ}_{11} = {S}_{11} ≅ {Γ}_{22} = {S}_{22}$ (1)

This approximation implies that the multiple reflection effects at the interfaces D1 and D2 are negligible for the high attenuation of the radiations through the liquid.

Considering the reflection coefficients Γ₁₁ and Γ₂₂, associated with two different experimental measures, the impedance (step 113) can be calculated based on the relationships: $\left\{\begin{matrix}{Z}_{d 11} = {Z}_{a} \frac{1 + {Γ}_{11}}{1 - {Γ}_{11}} \\ {Z}_{d 22} = {Z}_{a} \frac{1 + {Γ}_{22}}{1 - {Γ}_{22}}\end{matrix}\right.$ (4) wherein *Zₐ* is the impedance of an equivalent empty waveguide.

The impedance is closely related to the dielectric parameters, such as the relative dielectric permittivity εᵣ and the loss tangent tanδ, based on the relationship ${Z}_{d} = \frac{{ζ}_{0}}{\sqrt{{ε}_{r}} \sqrt{{Δ}_{d} - j tan δ}}$ (5) wherein ζ₀ is the impedance of vacuum, while Δ*_{d}* is the dispersion factor of the waveguide 9 given by ${Δ}_{d} = 1 - \frac{1}{{ε}_{r}} {\left(\frac{c}{2 af}\right)}^{2}$ (6) wherein c is the speed of light in the vacuum, a is the major side of the waveguide section and f is the frequency.

Using polar notation, the impedance is represented as ${Z}_{dii} = \left|{Z}_{dii}\right| {e}^{{jφ}_{ii}}$ (7) wherein |*Z_{dii}*| is the modulus, *φᵢᵢ* the step and *ii*=11 or *ii=22.*

By combining the relationship (5) and (7), it is possible to directly calculate the relative dielectric permittivity εᵣ and the loss tangent tanδ (step 107) from the impedance, according to the following relationships $\left\{\begin{matrix}{ε}_{rii} = {\left(\frac{c}{2 af}\right)}^{2} + \frac{{{ζ}_{0}}^{2}}{{\left|{Z}_{dii}\right|}^{2} \sqrt{1 + {tan}^{2} \left(2{φ}_{ii}\right)}} \\ {tanδ}_{ii} = {Δ}_{d} tan \left(2{φ}_{ii}\right)\end{matrix}\right.$ (8)

Next to the solutions of the inverse problem in approximate form, it is always possible to calculate the dielectric parameters by solving the full inverse problem, as shown in Figure 9. According to this process, the effects of the multiple reflections are not negligible, and therefore it is not allowed to assume the reflection coefficients Γ at the interfaces D1 and D2 equivalent to the actual reflection scattering parameters (S₁₁ and S₂₂).

For this reason, the step of calculating the actual scattering parameters (step 111) is followed by the analytical calculation of the reflection coefficient Γ and of the propagation factor P (step 114), given by $\left\{\begin{matrix}{Γ}_{11} = Q \left({S}_{11}, {S}_{21}\right) \pm \sqrt{1 + {Q}^{2} \left({S}_{11}, {S}_{21}\right)} \\ {P}_{21} = Q \left({S}_{21}, {S}_{11}\right) \pm \sqrt{1 + {Q}^{2} \left({S}_{21}, {S}_{11}\right)}\end{matrix}\right.$ (9) starting from the actual scattering parameters S₁₁ and S₂₁, and given by $\left\{\begin{matrix}{Γ}_{22} = Q \left({S}_{22}, {S}_{12}\right) \pm \sqrt{1 + {Q}^{2} \left({S}_{22}, {S}_{12}\right)} \\ {P}_{12} = Q \left({S}_{12}, {S}_{22}\right) \pm \sqrt{1 + {Q}^{2} \left({S}_{12}, {S}_{22}\right)}\end{matrix}\right.$ (10) starting from the actual scattering parameters S₂₂ and S₁₂, wherein *Q*(*S*₁₁, *S*₂₁) and, similarly *Q*(*S*₂₁, *S*₁₁), *Q*(*S*₂₂, *S*₁₂), *Q*(*S*₁₂, *S*₂₂), corresponds to $Q \left({S}_{11}, {S}_{21}\right) = \frac{1 + {{S}_{11}}^{2} + {{S}_{21}}^{2}}{2 {S}_{11}}$ (11)

Among the solutions of (9) and of (10), the one in which the transmission coefficient Γ and the propagation factor P are in a modulus less than one is selected, under the assumption of passivity.

The propagation factor P being known, it follows the step 115 of determining the constants of attenuation α and of propagation β, which are linked to the propagation factor P by the relationship $P = P \left(β, α\right) = {e}^{- {jγL}_{d}} = {e}^{- {αL}_{d}} {e}^{- {jβL}_{d}}$ (12)

Thus, the attenuation constant α is calculated based on $α = - \frac{ln \left|P\right|}{{L}_{d}}$ (13)

Whereas the propagation constant β is given by $β = {β}_{n} = {β}_{0} \pm \frac{2 πn}{{L}_{d}}$ (14) wherein *β*₀ is the baseband propagation constant and *n* is an integer.

*α* and *β*₀ being known, but not *β* given the undetermination of *n*, it is proceeded with step 107 relating to the calculation of the relative dielectric permittivity εᵣ and of the loss tangent tanδ of sample 4. In fact, εᵣ and tanδ are linked to α and β according to the following analytical expressions $\left\{\begin{matrix}β = {k}_{0} \sqrt{{ε}_{r} {Δ}_{d}} \left[\sqrt[4]{1 + {\left(\frac{tan δ}{{Δ}_{d}}\right)}^{2}}\right] cos ψ \\ α = β tan ψ\end{matrix}\right.$ (15) wherein *k₀=ω*/*c* is the propagation constant in an empty waveguide (*ω=2πf* is the pulsation and f the frequency), Δ*_{d}* is the dispersion factor defined in equation (6), whereas *ψ* is given by $ψ = \frac{1}{2} {tan}^{- 1} \left(\frac{tan δ}{{Δ}_{d}}\right)$ (16)

By rewriting the two relationships in (15) and also by inserting the equation (14), the following relationships are obtained which represent the conclusive equations to solve the full inverse problem $\left\{\begin{matrix}{α}^{2} + {β}^{2} = {k}_{0}^{2} {ε}_{r} {Δ}_{d} \sqrt{1 + {\left(\frac{tan δ}{{Δ}_{d}}\right)}^{2}} = {k}_{0}^{2} {ε}_{r} {Δ}_{d} \sqrt{1 + {\left(τ\right)}^{2}} \\ β = α \frac{\sqrt{1 + {τ}^{2}} + 1}{τ} = α \frac{\sqrt{1 + {\left(\frac{tan δ}{{Δ}_{d}}\right)}^{2}} + 1}{\frac{tan δ}{{Δ}_{d}}} \\ β = {β}_{n} = {β}_{0} \pm \frac{2 πn}{{L}_{d}}\end{matrix}\right.$ (17) wherein *εᵣ*, tan*δ* and *n* are the incognita

One of the possible ways to solve the equations of (17) and thus obtain *εᵣ* and tan*δ* is to apply an iterative process (step 116), starting for example from the relative dielectric permittivity εᵣ and from loss tangent tanδ which are calculated by solving the inverse problem in approximate form and which are given by the system relationships (8).

A verification on the solutions obtained at the end of each iteration consists in comparing the group delay derived experimentally (see Figure 10 and equation (1)) and the one derived analytically on the basis of the solutions obtained from the iteration.

The experimental group delay is calculated as ${τ}_{g 1} = {τ}_{g 21} - \frac{{τ}_{g 11} + {τ}_{g 22}}{2} ; {τ}_{g 2} = {τ}_{g 12} - \frac{{τ}_{g 11} + {τ}_{g 22}}{2}$ (18)

Whereas the analytical group delay is given by ${τ}_{g} = \frac{{L}_{d}}{{v}_{g}} = \frac{{L}_{d} ω}{{c}^{2}} \frac{{ε}_{r}}{β} \frac{\left(1+\frac{α}{β}tanδ\right)}{\left(1+{\left(\frac{α}{β}\right)}^{2}\right)}$ (19) wherein *v_{g}* is the group speed.

If the experimental and analytical group delay are equal, except for one tolerance, the calculated relative dielectric constant εᵣ and the calculated loss tangent tanδ are acceptable. Otherwise, a subsequent iteration is necessary, varying *n* twice, by one unit more and one unit less, in the third equation of the system (17), at the end of which two corresponding solutions for group delay are obtained. If these solutions are more discordant, it is concluded that the solutions of the previous iteration are acceptable. Otherwise, it is proceeded with a further iteration by varying *n* until the check of the values of the group delay at iteration with *n* + 1 and at iteration with *n -* 1 are worse than the iteration with *n*.

### EXAMPLES

### 1. Test case for the validation of the dielectric characterization of the sample

Below is a test case for the verification and validation of the dielectric characterization using the detection system of Figure 1 and solving the inverse problem both in approximate form and in full form for a sample 4 consisting of an isotonic buffer solution.

Based on well-known Debye model of water at room temperature (see W. Ellison, "Permittivity of Pure Water, at Standard Atmospheric Pressure, over the Frequency Range 0-25 THz and the Temperature Range 0-100 °C," Journal of Physical and Chemical Reference Data Vol. 36. No. 1, 2007), it is possible to preliminary hypothesize the range of values in which the relative dielectric permittivity εᵣ and the loss tangent tanδ of the measured isotonic buffer solution will be comprised relatively to the frequency band in the microwaves between 26 and 40 GHz. Specifically, the relative dielectric permittivity εᵣ of the measured isotonic buffer solution must be between 20 and 30, while the loss tangent tanδ must range from 1.2 to 1.4.

Figures 11 and 12 show, respectively, the trends of the reflection (S₁₁) and transmission (S₂₁) scattering parameters, acquired by the VNA 2. The oscillations, more associated with the reflection scattering parameter, are due to the geometric discontinuities in the sample holder, including the containment elements and the grooves in the fixing flange. Figures 11 and 12 show raw data (RawMeas) and filtered data (Win&Gated). The latter are filtered through a time gating technique in a time window *Δt* of about 0.7 ns, corresponding to 10 times the time resolution given by the bandwidth equal to 14 GHz in a WR28 waveguide and a spatial resolution *Δz =* c *Δt* of about 210 mm (where c is the speed of light).

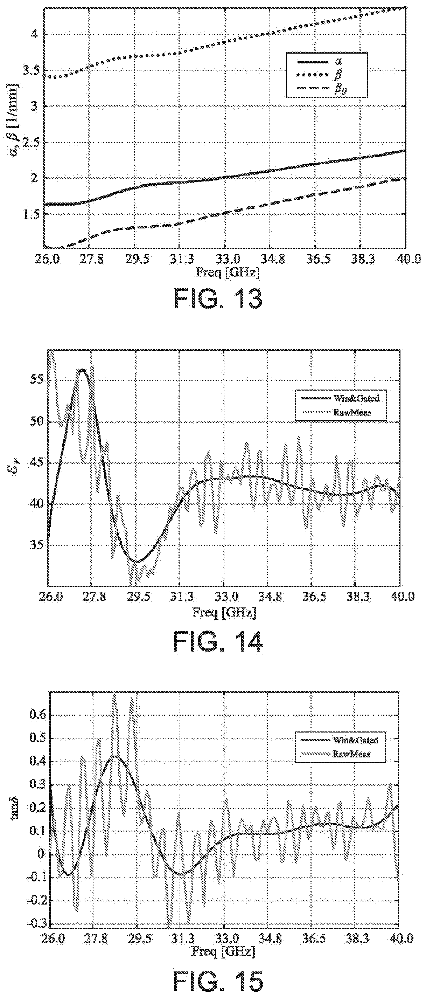

The trend of the attenuation constant α, of the propagation constant β and of the baseband propagation constant β₀ is shown in Figure 13. It can be noted that β₀ does not coincide with β, but β is given by the third equation of the system (17) with n equal to 1. The effects due to the geometric discontinuities are also present in the curves of Figure 13.

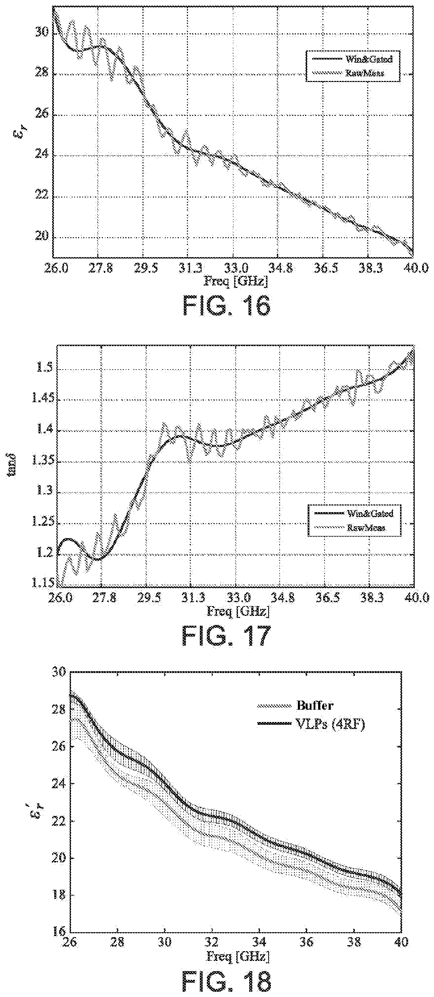

Figures 14 and 15 show, respectively, the trend of the relative permittivity εᵣ and of the loss tangent tanδ, which are obtained by solving the inverse problem in approximate form. It can be noted that the values of both the relative dielectric permittivity εᵣ and of the loss tangent tanδ are not included in the intervals hypothesized according to the Debye model. However, both solutions are used as initial estimates in the iterative process to solve the full inverse problem. The solutions, indicated in Figures 16 and 17, reached convergence at the end of the first iteration.

Taking into account the measured and analytical group delay, the validity of the solutions obtained with the full model was checked (the error between the measured and analytical group delay is approximately 70%). It should be noted that the values of the relative dielectric permittivity εᵣ (Figure 16) and the loss tangent tanδ (Figure 17) are in fact included in the ranges hypothesized based on Debye model.

By calculating the lengths L1 and L2 of the two extra portions, L1=0.2 mm and L2=0.16 mm were obtained, thus confirming the non-equivalence between the thickness of the containment elements (equal to 0.05 mm) and the lengths L1 and L2 of the extra portions.

### 2. Example of the method according to the invention

An application example of the method according to the present invention is reported here.

The reference solution of the first measurement 101 (indicated as "Buffer" in Figure 18) is an isotonic buffer solution with the addition of 1% BSA (Bovine Serum Albumin) and 0.05% Sodium azide.

The solution of the second measurement 102 (indicated as "VLPs (4RF)" in Figure 18) is given by the reference solution, as specified above, to which a specific concentration of VLP of the HIV/SIV virus equal to 1000 particles of VLP per µl of solution is added.

The volume of both the "Buffer" solution of the first measurement 101 and of the "VLPs (4RF)" solution of the second measurement 102 is equal to 76 µl.

Figure 18 shows the trend of the real part of the relative dielectric permittivity εᵣ' for both the "Buffer" solution and the "VLPs (4RF)" solution as the frequencies vary, calculated by the data processing processes detailed previously in the test case. In detail, the two curves of Figure 18 show the average value of the permittivity as a function of frequency and the uncertainty bars (standard deviation) due to the measurement error in a series of about 10 consecutive experiments, with regard to both the "Buffer" solution and the "VLPs (4RF)" solution. It can be observed that the curve relating to the "VLPs (4RF)" solution shows, throughout the bandwidth, substantially higher permittivity values than those relating to the buffer solution. These differences are therefore attributable to the presence of VLP in the solution "VLPs (4RF)".

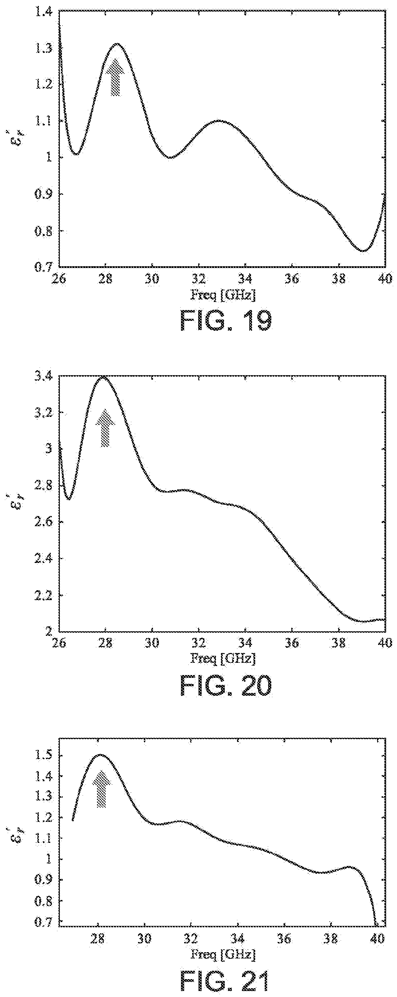

These differences are more highlighted in Figure 19, whose curve, properly called contrast, represents the difference between the permittivity values of the "VLPs (4RF)" solution and the buffer one. Thus, the curve of Figure 19 provides dielectric characterization of the solution with VLP.

The curve of Figure 19 also shows how there are frequencies in the working band 26-40 GHz at which the contrast between the trends of the permittivity of the two solutions is maximized; for example, 28 GHz is a frequency in which the difference in the real part of the dielectric permittivity between the buffer solution and the "VLP (4RF)" solution can be more appreciated.

The good repeatability of the results is confirmed by Figures 20 and 21. The contrasts shown in Figures 20 and 21 are in fact obtained starting from experimental data collected during different sessions of experiments. Similar to the contrast of Figure 19, both contrasts in Figures 20 and 21 show the maximum peak at 28 GHz frequency.

In practice, it has been found that the method of the invention achieves the intended aim.

In particular, the method of the invention allows detecting viral agents by dielectric spectroscopy in the microwaves with no need to introduce markers as in the case of PCR-based techniques.

## Claims

1. A method for detecting a viral agent comprising the steps of:
a) placing a first sample (4) comprising an aqueous solution into a non-resonance waveguide (9), which is axially delimited by a pair of containment elements (12) that are substantially transparent to the microwaves and define respective interfaces of the sample;
b) transmitting a signal with frequency variable in a predetermined microwave band to the first sample (4);
c) acquiring at least one dielectric parameter of the first sample (4) as the frequency varies by means of transmission and reflection measurements;
d) placing a second sample (4) into the waveguide(9);
e) transmitting said signal to the second sample (4);
f) acquiring the corresponding dielectric parameter of the second sample (4) as the frequency varies by means of transmission and reflection measurements; and
g) performing a differential spectroscopic analysis on said parameters for assessing the presence of a viral agent in at least one of said first sample (4) and second sample (4).

2. Method as claimed in claim 1, wherein the band range is 26 GHz to 40 GHz.

3. Method as claimed in claim 1 or 2, wherein the c) and f) steps comprise measuring transmission and reflection scattering parameters.

4. Method as claimed in claim 3, wherein steps b), c), e) and f) are implemented by means of a vector network analyser (2) comprising at least two ports connected to respective ports of the waveguide.

5. Method as claimed in claim 3 or 4, wherein step c) and f) comprise calculating said dielectric parameters based on the measured scattering parameters.

6. Method as claimed in claim 5, wherein calculating the dielectric parameters of the first sample and the second sample comprises the step of determining the actual reflection (Sii and Sjj) and transmission (Sij and Sji) scattering parameters at the interfaces of the samples starting from the measured reflection (sᵢᵢ, sⱼⱼ) and transmission (sᵢⱼ and sⱼᵢ) scattering parameters measured based on a model wherein the containment elements are represented by empty portions of the waveguide.

7. Method as claimed in claim 6, wherein calculating the dielectric parameters comprises the steps of:
• determining a reflection coefficient Γ at the sample interfaces and a propagation factor P of the sample;
• determining an attenuation constant and a propagation constant of the sample;
• deriving a relative dielectric permittivity (εᵣ) and a loss tangent (tanδ) of the sample.

8. Method as claimed in claims 6 and 7, wherein calculating the dielectric parameters comprises the step of calculating a sample impedance assuming a reflection coefficient Γ at the sample interferences equivalent to the actual reflection scattering parameters.

9. Method as claimed in one of the previous claims, wherein the first sample is an isotonic buffer solution, preferably a phosphate-buffered saline.

10. A system for the detection of a viral agent according to the method claimed in one of the previous claims, comprising a vector network analyser (VNA) (2) equipped with at least two ports (P1, P2), a sample holder (3) and two transmission lines (5) connecting the ports (P1, P2) of the VNA (2) to the sample holder (3), wherein the sample holder (3) is provided with a through cavity (8) configured to contain a sample (4) to be tested and defining a non-resonant waveguide (9), and comprises a pair of containment elements (12) that are substantially transparent and delimit the cavity (8) axially.

11. System as claimed in claim 10, wherein the waveguide (9) has a rectangular cross section.

12. System as claimed in claim 10 or 11, wherein the waveguide (9) is a WR28 type operating in a frequency band ranging from 26 GHz to 40 GHz.

13. System as claimed in one of the claims from 10 to 12, wherein the containment elements (12) comprise adhesive films.

14. System as claimed in one of the claims from 10 to 13, wherein the sample holder (3) comprises a perimeter flange (10) for connection to the transmission lines (5).

15. System as claimed in claim 14, wherein the transmission lines (5) comprise respective coaxial cables and respective coaxial cable/waveguide adapters (13).

16. System as claimed in one of the claims from 10 to 15, wherein the cavity (8) has an axial length ranging from 2 to 5 mm.

## Patentansprüche

1. Verfahren zum Nachweis eines viralen Erregers, umfassend die Schritte:
a) Einbringen einer ersten Probe (4), die eine wässrige Lösung umfasst, in einen nichtresonanten Wellenleiter (9), der axial durch ein Paar von für Mikrowellen im Wesentlichen durchlässigen Aufnahmeelementen (12) begrenzt ist, die jeweilige Grenzflächen der Probe definieren;
b) Übertragen eines Signals mit einer in einem vorgegebenen Mikrowellenband variierbaren Frequenz an die erste Probe (4);
c) Erfassen mindestens eines dielektrischen Parameters der ersten Probe (4) bei variierender Frequenz mittels Transmissions- und Reflexionsmessungen;
d) Einbringen einer zweiten Probe (4) in den Wellenleiter (9);
e) Übertragen des Signals an die zweite Probe (4);
f) Erfassen des entsprechenden dielektrischen Parameters der zweiten Probe (4) bei variierender Frequenz mittels Transmissions- und Reflexionsmessungen; und
g) Durchführen einer differentiellen spektroskopischen Analyse an den Parametern zur Beurteilung des Vorhandenseins eines viralen Erregers in mindestens einer der ersten Probe (4) und der zweiten Probe (4).

2. Verfahren nach Anspruch 1, wobei der Bandbereich 26 GHz bis 40 GHz beträgt.

3. Verfahren nach Anspruch 1 oder 2, wobei die Schritte c) und f) das Messen von Transmissions- und Reflexionsstreuparametern umfassen.

4. Verfahren nach Anspruch 3, wobei die Schritte b), c), e) und f) mittels eines Vektornetzwerkanalysators (2) durchgeführt werden, der mindestens zwei Anschlüsse umfasst, die mit entsprechenden Anschlüssen des Wellenleiters verbunden sind.

5. Verfahren nach Anspruch 3 oder 4, wobei Schritt c) und f) das Berechnen der dielektrischen Parameter auf Grundlage der gemessenen Streuparameter umfasst.

6. Verfahren nach Anspruch 5, wobei das Berechnen der dielektrischen Parameter der ersten Probe und der zweiten Probe den Schritt des Bestimmens der tatsächlichen Reflexions- (Sii und Sjj) und Transmissions- (Sij und Sji) Streuparameter an den Grenzflächen der Proben ausgehend von den gemessenen Reflexions- (Sii, Sjj) und Transmissions- (Sᵢⱼ und Sⱼᵢ) Streuparametern, die auf Grundlage eines Modells gemessen werden, umfasst, wobei die Aufnahmeelemente durch leere Abschnitte des Wellenleiters dargestellt werden.

7. Verfahren nach Anspruch 6, wobei das Berechnen der dielektrischen Parameter die folgenden Schritte umfasst:
- Bestimmen eines Reflexionskoeffizienten 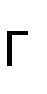 an den Probengrenzflächen und eines Ausbreitungsfaktors P der Probe;
- Bestimmen einer Dämpfungskonstante und einer Ausbreitungskonstante der Probe;
- Ableiten einer relativen dielektrischen Permittivität (εᵣ) und einer Verlusttangente (tanδ) der Probe.

8. Verfahren nach Anspruch 6 und 7, wobei das Berechnen der dielektrischen Parameter den Schritt des Berechnens einer Probenimpedanz unter Annahme eines Reflexionskoeffizienten 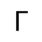 bei den Probeninterferenzen, die den tatsächlichen Reflexionsstreuparametern entsprechen, umfasst.

9. Verfahren nach einem der vorstehenden Ansprüche, wobei die erste Probe eine isotonische Pufferlösung, vorzugsweise eine phosphatgepufferte Kochsalzlösung, ist.

10. System zum Nachweis eines viralen Erregers nach dem Verfahren nach einem der vorhergehenden Ansprüche, umfassend einen Vektornetzwerkanalysator (VNA) (2), der mit mindestens zwei Anschlüssen (P1, P2), einem Probenhalter (3) und zwei Übertragungsleitungen (5) ausgestattet ist, die die Anschlüsse (P1, P2) des VNA (2) mit dem Probenhalter (3) verbinden, wobei der Probenhalter (3) mit einem durchgehenden Hohlraum (8) versehen ist, der ausgelegt ist, um eine zu testende Probe (4) aufzunehmen und einen nichtresonanten Wellenleiter (9) definiert, und ein Paar von Aufnahmeelementen (12) umfasst, die im Wesentlichen durchlässig sind und den Hohlraum (8) axial begrenzen.

11. System nach Anspruch 10, wobei der Wellenleiter (9) einen rechteckigen Querschnitt aufweist.

12. System nach Anspruch 10 oder 11, wobei der Wellenleiter (9) ein WR28-Typ ist, der in einem Frequenzband im Bereich von 26 GHz bis 40 GHz arbeitet.

13. System nach einem der Ansprüche 10 bis 12, wobei die Aufnahmeelemente (12) Klebefolien umfassen.

14. System nach einem der Ansprüche 10 bis 13, wobei der Probenhalter (3) einen umlaufenden Flansch (10) zur Verbindung mit den Übertragungsleitungen (5) umfasst.

15. System nach Anspruch 14, wobei die Übertragungsleitungen (5) jeweilige Koaxialkabel und jeweilige Koaxialkabel/Wellenleiter-Adapter (13) umfassen.

16. System nach einem der Ansprüche 10 bis 15, wobei der Hohlraum (8) eine axiale Länge im Bereich von 2 bis 5 mm aufweist.

## Revendications

1. Procédé de détection d'un agent viral, comprenant les étapes suivantes :
a) le placement d'un premier échantillon (4) comprenant une solution aqueuse dans un guide d'ondes de non-résonance (9), qui est délimité axialement par une paire d'éléments de confinement (12) étant sensiblement transparents aux micro-ondes et définissant des interfaces respectives de l'échantillon ;
b) la transmission au premier échantillon (4) d'un signal dont la fréquence varie dans une bande de micro-ondes prédéterminée ;
c) l'acquisition d'au moins un paramètre diélectrique du premier échantillon (4) au fur et à mesure que la fréquence varie, au moyen de mesures de transmission et de réflexion ;
d) le placement d'un second échantillon (4) dans le guide d'ondes (9) ;
e) la transmission dudit signal au second échantillon (4) ;
f) l'acquisition du paramètre diélectrique correspondant du second échantillon (4) au fur et à mesure que la fréquence varie, au moyen de mesures de transmission et de réflexion ; et
g) la réalisation d'une analyse spectroscopique différentielle sur lesdits paramètres pour évaluer la présence d'un agent viral dans au moins l'un desdits premier échantillon (4) et second échantillon (4).

2. Procédé selon la revendication 1, la plage de bande étant comprise entre 26 et 40 GHz.

3. Procédé selon la revendication 1 ou 2, les étapes c) et f) comprenant la mesure des paramètres de dispersion de transmission et de réflexion.

4. Procédé selon la revendication 3, les étapes b), c), e) et f) étant mises en œuvre au moyen d'un analyseur de réseau vectoriel (2) comprenant au moins deux ports connectés à des ports respectifs du guide d'ondes.

5. Procédé selon la revendication 3 ou 4, les étapes c) et f) comprenant le calcul desdits paramètres diélectriques sur la base des paramètres de dispersion mesurés.

6. Procédé selon la revendication 5, le calcul des paramètres diélectriques du premier échantillon et du second échantillon comprenant l'étape consistant à déterminer les paramètres de dispersion réels de réflexion (Sii et Sjj) et de transmission (Sij et Sji) aux interfaces des échantillons à partir des paramètres de dispersion de réflexion (Sii, Sjj) et de transmission (Sij et Sji) mesurés sur la base d'un modèle dans lequel les éléments de confinement sont représentés par des parties vides du guide d'ondes.

7. Procédé selon la revendication 6, le calcul des paramètres diélectriques comprenant les étapes consistant à :
- déterminer un coefficient de réflexion I aux interfaces d'échantillon et un facteur de propagation P de l'échantillon ;
- déterminer une constante d'atténuation et une constante de propagation de l'échantillon ;
- déduire une permittivité diélectrique relative (r) et une tangente de perte (tand) de l'échantillon.

8. Procédé selon les revendications 6 et 7, le calcul des paramètres diélectriques comprenant l'étape consistant à calculer une impédance d'échantillon en supposant un coefficient de réflexion I aux interfaces d'échantillon équivalent aux paramètres réels de dispersion de réflexion.

9. Procédé selon l'une des revendications précédentes, le premier échantillon étant une solution tampon isotonique, de préférence une solution saline tamponnée au phosphate.

10. Système de détection d'un agent viral selon le procédé revendiqué dans l'une des revendications précédentes, comprenant un analyseur de réseau vectoriel (VNA) (2) équipé d'au moins deux ports (P1, P2), un porte-échantillon (3) et deux lignes de transmission (5) reliant les ports (P1, P2) du VNA (2) au porte-échantillon (3), le porte-échantillon (3) étant pourvu d'une cavité traversante (8) configurée pour contenir un échantillon (4) à tester et définissant un guide d'ondes de non-résonance (9), et comprenant une paire d'éléments de confinement (12) qui sont sensiblement transparents et délimitent la cavité (8) axialement.

11. Système selon la revendication 10, le guide d'ondes (9) présentant une section transversale rectangulaire.

12. Système selon la revendication 10 ou 11, le guide d'ondes (9) étant de type WR28 fonctionnant dans une bande de fréquences allant de 26 à 40 GHz.

13. Système selon l'une des revendications de 10 à 12, les éléments de confinement (12) comprenant des films adhésifs.

14. Système selon l'une des revendications de 10 à 13, le porte-échantillon (3) comprenant une bride périphérique (10) destinée à être raccordée aux lignes de transmission (5).

15. Système selon la revendication 14, les lignes de transmission (5) comprenant respectivement des câbles coaxiaux et des adaptateurs de câble coaxial/de guide d'ondes (13).

16. Système selon l'une des revendications de 10 à 15, la cavité (8) présentant une longueur axiale comprise entre 2 et 5 mm.
